Europäisches Patentamt

European Patent Office

Office européen des brevets

(19)

(11) Publication number: **0 096 095**
**B1**

## (12) EUROPEAN PATENT SPECIFICATION

(45) Date of publication of patent specification: **09.11.88**

(51) Int. Cl.⁴: **G 01 N 33/483, G 01 N 27/12**

(21) Application number: **82105162.0**

(22) Date of filing: **14.06.82**

(54) **Semiconductor device, sensor and method for determining the concentration of an analyte in a medium.**

(43) Date of publication of application:
**21.12.83 Bulletin 83/51**

(45) Publication of the grant of the patent:
**09.11.88 Bulletin 88/45**

(84) Designated Contracting States:
**BE CH DE FR GB IT LI LU NL SE**

(56) References cited:
EP-A-0 036 274
DE-A-2 711 270
FR-A-2 096 026
FR-A-2 383 440
US-A-3 799 743
US-A-4 237 721

(73) Proprietor: **Ohmicron Corporation**
**108 West Franklin Ave.**
**Pennington, N.J. 08534 (US)**
(84) **BE CH DE FR GB IT LI LU NL SE**

(72) Inventor: **Ohmicron Corporation**
**108 West Franklin Ave.**
**Pennington, N.J. 08534 (US)**

(74) Representative: **Görtz, Dr. Fuchs, Dr.**
**Luderschmidt Patentanwälte**
**Sonnenberger Strasse 100 Postfach 26 26**
**D-6200 Wiesbaden (DE)**

The file contains technical information
submitted after the application was filed and
not included in this specification

Courier Press, Leamington Spa, England.

## Description

### Background of the invention

Immunoassays have been used routinely for the identification and quantitation of haptens, antigens and antibodies (all broadly termed *analytes*). The basic principle of all immunoassays is predicated on the specific binding between components of a reaction pair (e.g. antigen/antibody, hapten/antibody, etc.) where, in some cases, one component is labelled in such a fashion as to be easily analyzed by some external means.

Radioimmunoassay (RIA) is based on the use of a radioisotope as a label for one of the components of a specific binding pair. A radioisotopically labelled component can then be detected by counting the radiation emitted by the isotope using a suitable instrument.

Other methods of labelling one component of a specific binding pair have been developed. The use of enzyme and fluorescent labels have recently been employed and are termed enzyme immunoassay (EIA) and fluorescent immunoassay (FIA) respectively. Again, with the use of suitable reagents and instruments, these labels can be used for the determination of analytes in a liquid medium. Many variations to the basic procedures are in use, but most require the steps of reaction, separation, and detection of label.

More recently, electrochemical sensors have been employed in an effort to simplify and/or improve the sensitivity of these procedures. Basically, they employ an ion selective electrode to detect the reaction product of an enzyme which has been used as a level for one component of a specific binding pair.

The present invention seeks to eliminate the preparation of a labelled component of a specific binding pair, the separation of such a component from the assay system, as well as its subsequent detection; thereby greatly simplifying the method of performing an immunoassay. More specifically, the present invention relates to a new and useful improvement in a method for the determination of analytes in a liquid medium by the use of a biochemically sensitive semiconductor device set forth in the following description and specifications.

### Summary of the invention

The present invention relates to a device to be used in a method for the determination of analytes in a medium. More specifically, the present invention relates to a device, composed of an electrically semiconductive material suitably a semiconductive organic polymer as mentioned in claim 1 to which an analyte specific binding substance is suitably immobilized to said material in such a fashion that the binding of said analyte to its specific binding substance alters the electrical semiconductive properties of the semiconductive material in a measureable way. Further, the present invention relates to a method of determining the presence of an analyte in a medium using such a device.

US—A—3999122 mentions the use of semiconductors and lists certain reactive organic materials which are semiconductive and which can be used in a sensing device which has a layer comprising these materials; cartenoids, proteins, polypropylene, phthalocyanine, polyvinyl chlorides and cellulose acetate.

As most specific binding substances for any particular analyte are biological in origin, said device is termed a biochemically sensitive semiconductor device or BSSD. These specific binding substances are generally of an organic chemical nature, displaying certain measurable properties of which one is a specific electrical charge. Furthermore, for a specific binding substance, its electrical charge will vary as a result of its binding to its particular analyte; an example of a binding substance and its specific analyte is an antibody and its specific antigen. In accordance with this invention the binding of an antigen (analyte) by its specific antibody (binding substance) may be determined by detecting and measuring the change in the electrical charge of one or both elements of the binding reaction. By placing either one of the two elements of a specific binding system in close proximity to a material which can be influenced by the field of the electrical charge, a change in that electrical field as a result of the binding reaction will effect a change in the properties of that material. If the properties of this material are measurable, it follows that the binding reaction is also measureable.

### Brief description of the drawings

Figure 1 is a schematic cross-sectional elevational view of the element portion of the device.

Figure 2 is a further embodiment of Figure 1.

Figure 3 is a circuit diagram for operation of the device.

Figure 4 is a perspective plan view of an embodiment of the carrier portion of the device.

### Detailed description of the invention

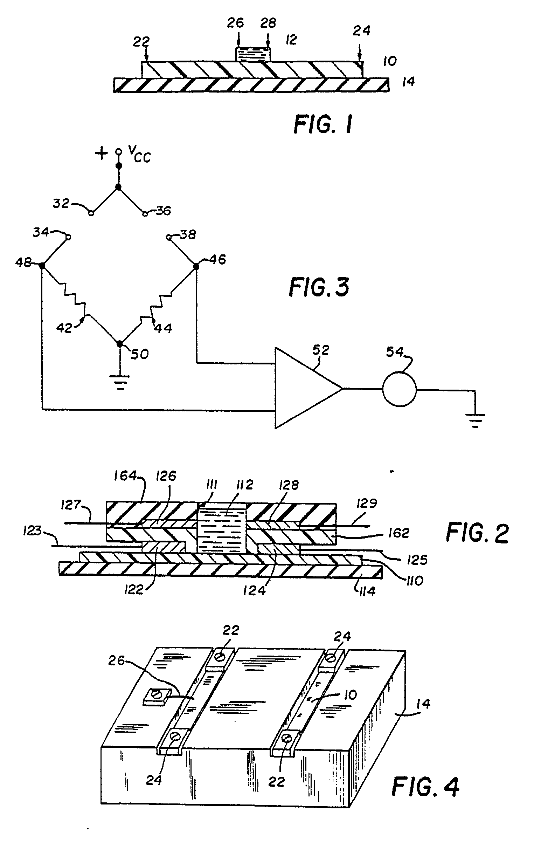

The invention and its operation may be best described by referring separately to the element portion as illustrated in Figures 1, 2 and 4 and the electrical operation of the device as illustrated in Figure 3.

The central element 10 of the device comprises a strip of semiconductive organic polymer. Any semiconductive organic polymer may be utilized. This rather novel class of compounds includes polyacetylene, poly(p-phenylene), polypyrrole, polyalkadiynes such as poly(hepta-1,6-dine), poly(p-phenylene sulfide), polymetallophthalocyanines, suitably polyaluminophthalocyanine fluoride and polyphthalocyanine siloxane.

The organic polymers set forth above may be utilized either in the undoped condition or containing conventionally employed dopants, for example, arsenic pentafluoride, iodine, perchlorate, and the like.

The element 10 is exposed to a binding agent. It is generally sufficient to soak the element in an aqueous solution of the binding agent in a suitable buffer. The term "binding agent" in this context is understood to include any material which will undergo a coupling reaction of the type generally understood in biochemistry to be of the antigen/antibody reaction.

Illustrative of binding agents are antibody to IgG, enzymes such as β-galactosidase, concanavalin A, limulin, cofactor type enzymes such as isocitrate dehydrogenase, alcohol dehydrogenase, NAD (nicotinamide adenine dinucleotide), enzyme substrates such as isocitrate enzymes inhibitors such as, β-galactosidase inhibitors hormones such as thyroxine, enzyme substrate analogs, such as σ-nitrophenyl-β-D-galactopyranoside antigens such as human chlorionic gonadotropin. Again these illustrations are not to be considered as critical or limiting.

While the illustrations of the present invention disclose the simple contact treatment of the binding agent with the polymeric element, it will be understood by those skilled in the art that under certain circumstances an activating treatment of the substrate of the element may be necessary to bind to certain binding agents, such reactions generally are conventional and are well known to those skilled in the art and are included within the scope of the present invention.

In the normal course of treatment of the element 10 with the binding agent, freshly synthesized polymer is placed in a suitable buffer containing the solution of the desired binding agent, incubated at room temperature from about 8 to about 24 hours, washed with saline solution and stored under nitrogen. While nitrogen storage is not entirely necessary it has been found that may binding agents are sensitive to oxygen and their activity is best preserved by reducing possible exposure to this substance.

The schematic representation of the central portion of the device is shown in Figure 1 wherein element 10 comprising the semiconductive material and the binding agent associated therewith are mounted on an insulating material 14. There are applied to element 10 electrical contacts 22 and 24. When the device is in operation and the analyte solution 12 is placed thereon, additional electrical contacts 26 and 28 are applicable to medium 12. This can be noted by reference to Figure 4 showing at the right hand portion thereof the location of contacts 22 and 24 in relationship to element 10 and insulating carrier 14. At the lefthand side thereof it is shown a similar arrangement showing contact 26. Contact 28 could be similarly applied. It is particularly preferred for reasons of good connection that the contacts be gold plated or of gold wire.

Where it is desired to utilize the full potential of the apparatus a slightly more complex arrangement as illustrated in Figure 2 may be employed. The last two digits of the item numbers correspond to the appropriate two digit numbers in Figure 1.

Thus, Figure 2 illustrates a base 114 carrying element 110 comprising the semiconductive organic polymer and the binding agent. Upon the other surface of the element 110 are placed a pair of metallic contacts 122 and 124 having the leads 123 and 125 respectively connected thereto. The lower portion of a well is created by disc shaped insulating elements 162 having the illustrated L shaped cross section. Over disc element 162 positioned to be on opposite sides of a well 111 are electrodes 126 and 128 having leads 127 and 129 respectively attached thereto. These contacts 126 and 128 comprise only a very small portion of the wall area of the well and are surrounded by insulating disc element 164 medium causing the analyte 112 is placed in the thus produced well 111.

The device is conventionally operated in the Wheatstone Bridge circuit illustrated in Figure 3. In this circuit a positive potential is applied between contact points 32 and 36. One element 10 is connected between contacts 32 and 34 and a similar element is connected between contacts 36 and 38. Contact 34 is connected to a junction 48 to which is connected one end of resistor 42 which may, if desired, be a variable resistor. Similarly, one end of another resistor 44 which may also be a variable resistor is connected at junction point 46 to which contact 38 is also connected. Junctions 46 and 48 are connected to a differential amplifier 52 which is connected to ground through a meter device 54.

In the operation of the device the two elements 10 connected to 32/34 and 36/38 respectively are balanced in the conventional manner. Medium containing the analyte is then placed, say, in well 111 and the change in resistance of that particular element is measured. It has been found that the change in resistance is a factor of time and concentration and thus calibration in a standard solution is desirable. Further it is possible to increase the sensitivity of the measurement by applying a potential to either contact 26 or contact 28 or, if the potential is equal, to both at the same time. If desired, contacts 26 and 28 may be utilized to measure the conductivity of the medium in a conventional manner.

The term "analyte" as used herein refers to antigens, antibodies, haptens, enzymes, enzymes substrates, enzyme substrates analogs, agglutinins, lectin, enzyme cofactors, enzyme inhibitors, hormones and the like.

Experimental
Preparation A

The IgG fraction of goat anti-rabbit IgG serum was isolated by a combination of 33% ammonium sulfate precipitation and DEAE cellulose chromatography as described by Garvey, J.S. et al (1980) in *Methods in Immunology,* pp. 193—198, W.A. Benjamin Inc. Further specific purification, as necessary was effected by the technique of affinity chromatography, various procedures of which are described in the literature.

**Preparation AA**

The trans-polymer of acetylene was prepared, using a Zeigler-type catalyst, following the procedure of Ito et al., Journal of Polymer Science, *12*, 11, (1974).

**Preparation BB**

Poly(p-phenylene) is prepared by the procedure of Shacklette et al Syn. Met. *1*, 307 (1980).

**Preparation CC**

Polypyrrole is prepared by the procedure of Kanazawa et al Syn. Met. *1*, 329 (1980).

**Preparation DD**

Poly(hepta-1,6-diyne) is prepared by the method of Gibson, et al., J. Chem. Soc. (Chem. Comm) *426*, (1980).

**Preparation EE**

Poly(p-phenylene sulfide) hexafluoroarsenate is prepared by the method of Rabolt et al, J. Chem. Soc. (Chem. Comm.) *347*, (1980).

**Example I**

Strips of freshly synthesized polyacetylene, approximately 4 mm×20 mm, were placed in a 0.05 M carbonte-bicarbonate buffer, pH 9.5 containing 5 mg/ml of purified goat anti-rabbit IgG and incubated overnight at room temperature. The polyacetylene antibody strips were subsequently washed with a saline solution and stored under nitrogen.

**Example II**

Strips of freshly synthesized poly(p-phenylene), approximately 4 mm×20 mm, are placed in a 0.05 M carbonate-bicarbonate buffer, pH 9.5 containing 5 mg/ml of monoclonal antibody to human chorionic ganodotropin and incubated overnight at room temperature. The poly(p-phenylene) strips are subsequently washed with a saline solution and stored under nitrogen.

**Example III**

Strips of freshly synthesized polypyrrole, approximately 4 mm×20 mm, are placed in a 0.05 M carbonate-bicarbonate buffer, pH 9.5 containing 5 mg/ml of avidin and incubated overnight at room temperature. The polypyrrole/avidin strips are subsequently washed with a saline solution and stored under nitrogen.

**Example IV**

Strips of freshly synthesized poly(hepta-1,6-diyne), approximately 4 mm×20 mm, are placed in a 0.05 M carbonate-bicarbonate buffer, pH 9.5 containing 5 mg/ml of β-galactosidase and incubated overnight at room temperature. The poly-(hepta-1,6-diyne)/β-galactosidase strips are subsequently washed with a saline solution and stored under nitrogen.

**Example V**

Strips of freshly synthesized poly(p-phenylene sulfide)hexafluoroarsenate, approximately 4 mm×20 mm are placed in a 0.05 M carbonate-bicarbonate buffer, pH 9.5 containing 5 mg/ml of limulin and incubated overnight at room temperature. The poly(p-phenylenesufide)hexafluoroarsenate/limulin strips are subsequently washed with a saline solution and stored under nitrogen.

**Example VI**

Strips of freshly synthesized polyaluminophthalocyanine fluoride, approximately 4 mm×20 mm, are placed in a 0.05 M carbonate-bicarbonate buffer, pH 9.5 containing 5 mg/ml of NAD and incubated overnight at room temperature. The polyaluminophthalocyanine fluoride strips are subsequently washed with a saline solution and stored under nitrogen.

**Example VII**

A pair of elements prepared in accordance with Example I but connected into a pair of devices in accordance with the right hand portion of the device illustrated in Figure 4 and connected into the circuit of Figure 2. The bridge circuit was brought into balance and 10 microliters of rabbit IgG in PBS buffer (500 micrograms of IgG/ml) were placed on one element and the same volume of PBS buffer on the other element taking care to avoid contact of either sample with the metallic contact elements 22 and 24 respectively. Element 44 in the circuit is a variable transformer and readings were taken from T=0 to T=1 hour as follows:

| T=0 | 470 |
| T=1 min | 498 |
| T=2 min | 488 |
| T=3 min | 563 |
| T=9 min | 731 |
| T=20 min | 687 |
| T=1 hr | 587 |

The supply of voltage was 5.1 volts.

The experiment was repeated using 20 microliters of IgG with a supply voltage of 5.0 volts and the following readings obtained.

| T=0 | 378 |
| T=1 | 358 |
| T=5 | 403 |
| T=10 | 499 |
| T=20 | 499 |
| T=30 | 435 |

**Example VIII**

As in Example I, a strip of polyacetylene-antibody was prepared, using a goat anti-rabbit IgG preparation, and placed in a teflon holder. A mesh with a 3 mm diameter cutout was placed over the polyacetylene film and used to overlay an aqueous gelatin film, taking the precaution not to physically or electrically connect the two conductive clamps with the gelatin film. A measurement of the specific analyte was made with this device as described in Example VII.

Example IX

As in Example VIII, a strip of polyacetylene-antibody gelating film was prepared except that a third electrical connection was formed by placing a piece of platinum wire just on the surface of the gelatin film. In this example, the device is similar in principle to a field effect transistor. This third electrical connection allows for the application of an electrical potential at a right angle to the flow of electrons through the polyacetylene-antibody film. Using this device, the binding of rabbit IgG to goat anti-rabbit IgG antibody was ascertained as described in Example VII.

The procedures of the proceeding Examples were repeated utilizing a device of Figure 2 wherein 2 to 10 volts are applied to contact 126 in the device.

The invention being thus described, it will be obvious that the same may be varied in many ways. Such variations are not to be regarded as a departure from the scope of the invention, and all such modifications are to be included within the scope of the following claims.

**Claims**

1. A variable resistance sensor for the determination of an analyte in a medium, comprising
   a) an insulating structure (14, 114)
   b) element means (10, 110) having an active part comprised of semiconductive organic polymer including a specific binding substance for the analyte, mounted on said structure (14, 114) for exposure to the medium (12, 112) and
   c) electrodes position to contact the element (10, 110)
   characterized in 'that said semiconductive organic polymer is selected from the group consisting of polyacetylene, poly(p-phenylene), polypyrrole, polyalkadiynes, poly(p-phenylene sulfides), polymetallophthalocyanines and polyphthalocyanine siloxane.

2. A sensor of Claim 1 characterized in that it additionally comprises a means (111) for bringing the analyte in the medium (112) in contact with the element (110).

3. A sensor in accordance with Claims 1 or 2 characterized in that the said polymer is polyacetylene.

4. A sensor in accordance with any of Claims 1 through 3 characterized in that there are two electrodes (22, 122, 24, 124).

5. A sensor in accordance with any of Claims 1 through 3 characterized in that there are three electrodes (22, 122, 24, 124, 26, 126).

6. A sensor in accordance with any of Claims 1 through 3 characterized in that there are four electrodes (22, 122, 24, 124, 26, 126, 28, 128).

7. A sensor in accordance with any of Claims 1 through 6 characterized in that the specific binding substance is selected from the group consisting of antibodies, antigens, enzymes, enzyme substrates, analogs of enzyme substrates, agglutinins, lectins, enzyme cofactors, enzyme inhibitors and hormones.

8. A method of identifying the type and quantity of an analyte in a medium comprising the steps of:
   a) preparing an element of semiconductor organic polymer,
   b) treating the element with a specific binding substance,
   c) exposing the element to the medium,
   d) measuring the change in an electrical characteristic of the treated element,
   characterized in that the semiconductive organic polymer is selected from the group consisting of polyacetylene, poly(p-phenylene), polypyrrole, polyalkadiynes, poly(p-phenylene sulfides), polymetallophthalocyanines and polyphthalocyanine siloxane.

9. A method in accordance with Claim 8 characterized in that the organic polymer is polyacetylene.

10. A method of Claims 8 or 9 characterized by the inclusion of the steps of applying at least two electrodes to contact the element and measuring the electrical changes between the electrodes.

11. A method of Claim 10 characterized by utilizing three electrodes to contact the element.

12. A method of Claim 11 characterized by applying four electrodes to contact the element.

13. A method of any of Claims 9 through 12 characterized in that the specific binding substance is selected from the group consisting of an antibody, an antigen, an enzyme, an enzyme substrate, an enzyme substrate analog, an agglutinin or lectin, an enzyme cofactor, an enzyme inhibitor and a hormone.

**Patentansprüche**

1. Meßfühler mit veränderbarem Widerstand zum Bestimmen eines Analyten in einem Medium mit
   a) einem nichtleitenden Träger (14, 114),
   b) einer auf dem Träger zum Aussetzen gegenüber dem Medium (12, 112) angebrachten Elementeinrichtung (10, 110) mit einem aktiven Teil aus einem halbleitenden organischen Polymer einschließlich eines spezifischen Bindemittels für den Analyten, und
   c) Elektroden, die das Element (10, 110) kontaktierend anordnen,
   dadurch gekennzeichnet, daß das halbleitende organische Polymer gewählt ist aud der Gruppe, bestehend aus Polyacetylen, Poly-(p-phenylen), Polypyrrol, Polyalkadiynen, Poly(p-phenylen-sulfiden), Polymetallophthalocyaninen und Polyphthalocyaninsiloxan.

2. Meßfühler nach Anspruch 1, dadurch gekennzeichnet, daß er weiterhin Mittel (111) zum Inkontaktbringen des Analyten im Medium (112) mit dem Element (110) aufweist.

3. Meßfühler nach den Ansprüchen 1 oder 2, dadurch gekennzeichnet, daß das Polymer Polyacetylen ist.

4. Meßfühler nach einem der Ansprüche 1—3, dadurch gekennzeichnet, daß zwei Elektroden (22, 122, 24, 124) vorhanden sind.

5. Meßfühler nach einem der Ansprüche 1—3, dadurch gekennzeichnet, daß drei Elektroden (22, 122, 24, 124, 26, 126) vorhanden sind.

6. Meßfühler nach einem der Ansprüche 1—3, dadurch gekennzeichnet, daß vier Elektroden (22, 122, 24, 124, 26, 126, 28, 128) vorhanden sind.

7. Meßfühler nach einem der Ansprüche 1—6, dadurch gekennzeichnet, daß das spezifische Bindemittel ausgewählt ist aus der Gruppe, bestehend aus Antikörpern, Antigenen, Enzymen, Enzymsubstraten, Enzymsubstratanalogen, Agglutininen, Lectinen, Enzymfaktoren, Enzyminhibitoren und Hormonen.

8. Verfahren zum Erkennen der Art und der Menge eines Analyten in einem Medium mit den Schritten:

a) Herstellen eines Elementes aus einem halbleitenden organischen Polymer,

b) Behandlung des Elementes mit einem spezifischen Bindemittel,

c) Aussetzen des Elementes gegenüber dem Medium,

d) Messen der Veränderung einer elektrischen Eigenschaft des behandelten Elementes,

dadurch gekennzeichnet, daß das halbleitende organische Polymer ausgewählt ist aus der Gruppe, bestehend aus Polyacetylen, Poly-(p-phenylen), Polypyrrol, Polyalkadiynen, Poly-(p-phenylensulfiden), Polymetallophthalocyaninen und Polyphthalocyanin- siloxan.

9. Verfahren nach Anspruch 8, dadurch gekennzeichnet, daß das organische Polymer Polyacetylen ist.

10. Verfahren nach Anspruch 8 oder 9, dadurch gekennzeichnet, daß man in weiteren Schritten wenigstens zwei das Element kontaktierende Elektroden einsetzt und die elektrischen Änderungen zwischen den Elektroden mißt.

11. Verfahren nach Anspruch 10, gekennzeichnet durch die Verwendung von drei das Element kontaktierenden Elektroden.

12. Verfahren nach Anspruch 11, gekennzeichnet durch die Anwendung von vier das Element kontaktierenden Elektroden.

13. Verfahren nach einem der Ansprüche 9—12, dadurch gekennzeichnet, daß das spezifische Bindemittel ausgewählt ist aus der Gruppe, bestehend aus einem Antikörper, einem Antigen, einem Enzym, einem Enzymsubstrat, einem Enzymsubstratanalogen, einem Agglutinin oder Lectin, einem Enzymfaktor, einem Enzyminhibitor und einem Hormon.

**Revendications**

1. Capteur à résistance variable pour déterminer un analyte dans un milieu, qui comprend:

a) un support isolant (14, 114),

b) un élément (10, 110) comportant une partie active formée d'un polymère organique semi-conducteur portant une substance de combinaison spécifique pour l'analyte, qui est monté sur le support (14, 114) pour l'exposition au milieu (12, 112), et

c) des électrodes positionnées pour venir au contact de l'élément (10, 110),

caractérisé en ce que le polymère organique semi-conducteur est choisi dans la classe formée par le polyacétylène, le poly(p-phénylène), le polypyrrole, les polyalcadiynes, les poly(sulfures de p-phénylène), les polymétallophtalocyanines et le polyphtalocyanine-siloxane.

2. Capteur suivant la revendication 1, caractérisé en ce qu'il comprend, en outre, un dispositif (111) pour amener l'analyte dans le milieu (112) en contact avec l'élément (110).

3. Capteur suivant la revendication 1 ou 2, caractérisé en ce que le polymère est le polyacétylène.

4. Capteur suivant l'une quelconque des revendications 1 à 3, caractérisé en ce qu'il y a deux électrodes (22, 122, 24, 124).

5. Capteur suivant l'une quelconque des revendications 1 à 3, caractérisé en ce qu'il y a trois électrodes (22, 122, 24, 124, 26, 126).

6. Capteur suivant l'une quelconque des revendications 1 à 3, caractérisé en ce qu'il y a quatre électrodes (22, 122, 24, 124, 26, 126, 28, 128).

7. Capteur suivant l'une quelconque des revendications 1 à 6, caractérisé en ce que la substance de combinaison spécifique est choisie dans la classe formée par les anticorps, antigènes, enzymes, substrats pour enzymes, analogues de substrats pour enzymes, agglutinines, lectines, cofacteurs d'enzymes, inhibiteurs d'enzymes et hormones.

8. Procédé pour identifier la nature et la quantité d'un analyte dans un milieu, qui comprend les stades:

a) de préparer un élément de polymère organique semi-conducteur,

b) de traiter l'élément avec une substance de combinaison spécifique.

c) d'exposer l'élément au milieu,

d) de mesurer la modification d'une caractéristique électrique de l'élément traité,

caractérisé en que le polymère organique semi-conducteur est choisi dans la classe formée par le polyacétylène, le poly(p-phénylène), le polypyrrole, les polyalcadiynes, les poly(sulfures de p-phénylène), les polymétallophtalocyanines et le polyphtalocyaninesiloxane.

9. Procédé suivant la revendication 8, caractérisé en ce que le polymère organique est le polyacétylène.

10. Procédé suivant la revendication 8 ou 9, caractérisé en ce qu'il comprend, en outre, les stades d'appliquer au moins deux électrodes pour venir au contact de l'élément et de mesurer les modifications électriques entre les électrodes.

11. Procédé suivant la revendication 10, caractérisé par l'utilisation de trois électrodes pour venir au contact de l'élément.

12. Procédé suivant la revendication 11, caractérisé par l'utilisation de quatre électrodes pour venir au contact de l'élément.

13. Procédé suivant l'une quelconque des revendications 9 à 12, caractérisé en ce que la substance de combinaison spécifique est choisie dans la classe formée par les anticorps, antigènes, enzymes, substrats pour enzymes, analogues de substrats pour enzymes, agglutinines, lectines, cofacteurs d'enzymes, inhibiteurs d'enzyme et hormones.

FIG. 1

FIG. 3

FIG. 2

FIG. 4